# EUROPEAN PATENT APPLICATION

(11) **EP 4 610 245 A1**
(43) Date of publication of application: **03.09.2025**
(21) Application number: 23882689.5
(22) Date of filing: 25.10.2023
(51) Int. Cl.: C07C 17/16

(54) **FLUORINE-CONTAINING COMPOUND PRODUCTION METHOD**

(30) Priority: 25.10.2022 JP 2022170509
(71) Applicant: AGC INC., Chiyoda-ku, Tokyo 1008405 (JP); The University of Tokyo, Bunkyo-ku, Tokyo 113-8654 (JP); Kyoto University, Kyoto-shi, Kyoto 606-8501 (JP)
(72) Inventor: AIKAWA, Kohsuke, Tokyo 113-8654 (JP); NOZAKI, Kyoko, Tokyo 113-8654 (JP); GATZENMEIER, Tim, Lincoln, Nebraska (US); HAGIWARA, Rika, Kyoto-shi, Kyoto 606-8501 (JP); MATSUMOTO, Kazuhiko, Kyoto-shi, Kyoto 606-8501 (JP); ISHIBASHI, Yuichiro, Tokyo 100-8405 (JP); KASHIWAGI, Kimiaki, Tokyo 100-8405 (JP); OKAZOE, Takashi, Tokyo 100-8405 (JP)
(74) Representative: Müller-Boré & Partner Patentanwälte PartG mbB
(86) International application number: PCT/JP2023/038549
(87) International publication number: WO 2024/090485

(57) **Abstract**

The present invention provides a method for producing a fluorine-containing compound by introducing a fluorine atom into a wide variety of oxygen-containing compounds having a ketone group, an aldehyde group, a hydroxy group, or the like using SF₅⁻ stabilized by forming a salt with a glyme-coordinated alkali metal ion as a deoxofluorinating agent under relatively mild conditions.

The present invention pertains to a method for producing a fluorine-containing compound, the method in which a fluorine-containing compound is produced by the deoxofluorination of an oxygen-containing compound having an aldehyde group, a ketone group, a carboxy group, or a hydroxy group in one or more solvents selected from the group consisting of DMPU, G4, DMA, HMPA, and MeCN using a deoxofluorinating agent represented by a general formula (E1) [in the formula, M represents Cs⁺, K⁺, or Rb⁺; G4 represents a tetraethylene glycol dimethyl ether; and n represents an integer of 1 to 4].
[Chemical Formula 1]

[M(G4)ₙ] [SF₅] (E1)

## Description

### TECHNICAL FIELD

The present invention relates to a method for producing a fluorine-containing compound by introducing a fluorine atom into an oxygen-containing compound having a ketone group, an aldehyde group, a hydroxy group, or the like using a deoxofluorinating agent.

Priority is claimed on Japanese Patent Application No. 2022-170509, filed October 25, 2022, the content of which is incorporated herein by reference.

### BACKGROUND ART

Fluorine atoms have high electronegativity, and are small like hydrogen atoms. Due to this characteristic, fluorine atoms can bond stably with many atoms, and organic compounds into which fluorine has been introduced tend to have improved heat resistance, chemical resistance, light resistance, water resistance, and the like compared to those prior to the introduction. In particular, carbon-fluorine bonds have a short bond distance, are rigid, and have low polarizability. Due to this characteristic of carbon-fluorine bonds, organic compounds into which carbon-fluorine bonds have been introduced have reduced reactivity and improved stability as compounds. Various fluorinating agents have been developed because useful organic compounds can be synthesized by fluorinating organic compounds.

For example, N-diethylaminosulfur trifluoride (DAST) is known as a fluorinating agent. For example, Non-Patent Document 1 reports that an acylpyrrole compound undergoes a deoxofluorination reaction with DAST by protecting an NH in a pyrrole ring, and a difluoroalkylpyrrolic compound is synthesized. Further, DAST functions as a deoxofluorinating agent also for carboxylic acids and aldehydes. For example, Patent Document 1 reports that DAST produces retinoyl fluoride from retinoic acid and 15, 15-difluoroaxerophthene from retinal, respectively. However, it is difficult to deoxofluorinate compounds having a free NH, such as acylpyrrole compounds, without protecting the NH.

In addition, SF₄ is also useful as a deoxofluorinating agent, but deoxofluorination with SF₄ requires the coexistence of HF. Accordingly, as a complex of SF₄ which is more stable and easier to use, Non-Patent Document 2 reports the use of a salt of a glyme-coordinated alkali metal ion and SF₅⁻ ([M(G4)n][SF₅]) as a deoxofluorinating agent. This document reports that a deoxofluorinated product was obtained by reacting 3-phenyl-1-propanol with [Cs(G4)₂][SF₅] in THF, but the yield was low and further improvement is required.

### Citation List

### Patent Document

Patent Document 1: U.S. Patent No. 4,473,503

### Non Patent Documents

Non Patent Document 1: Wang, et al., Tetrahedron Letters, 1995, vol. 36(14), p. 2389-2392.
Non Patent Document 2: Matsumoto, et al., Inorganic Chemistry, 2018, vol. 57, p. 14882-14889.

### SUMMARY OF INVENTION

### Technical Problem

The present invention aims to provide a method for producing a fluorine-containing compound by introducing a fluorine atom into a wide variety of oxygen-containing compounds having a ketone group, an aldehyde group, a hydroxy group, or the like using SF₅⁻ stabilized by forming a salt with a glyme-coordinated alkali metal ion as a deoxofluorinating agent under relatively mild conditions.

### Solution to Problem

The inventors of the present invention discovered that deoxofluorination using a salt of a glyme-coordinated alkali metal ion and SF₅⁻ as a deoxofluorinating agent in a specific solvent can deoxofluorinate a wide variety of oxygen-containing compounds under relatively mild conditions, thereby leading to completion of the present invention.

That is, the present invention is as follows.
[1] A method for producing a fluorine-containing compound, the method including deoxofluorinating an oxygen-containing compound having an aldehyde group, a ketone group, a carboxy group, or a hydroxy group in one or more solvents selected from the group consisting of 1,3-dimethyl-3,4,5,6-tetrahydro-2(1H)-pyrimidinone, tetraethylene glycol dimethyl ether, dimethylacetamide, hexamethylphosphorous triamide, and acetonitrile, using a deoxofluorinating agent represented by the following general formula (E1):
   [Chemical Formula 1]

   [M(G4)ₙ] [SF₅] (E1)

   [in the formula, M represents a cesium ion, a potassium ion, or a rubidium ion; G4 represents tetraethylene glycol dimethyl ether; and n represents an integer from 1 to 4] to produce a fluorine-containing compound.
[2] The method for producing a fluorine-containing compound according to [1] above, wherein the aforementioned oxygen-containing compound is a compound represented by the following general formula (S1): 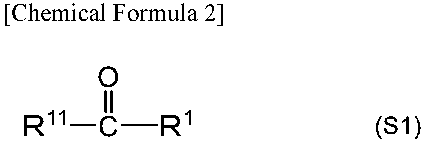
   [in the formula, R¹ is a hydrogen atom or an alkyl group having 1 to 6 carbon atoms; and R¹¹ is an aliphatic hydrocarbon group having 1 to 30 carbon atoms which may have a substituent, an alkoxy group which may have a substituent, an aliphatic heterocyclic group which may have a substituent, an aryl group which may have a substituent, or a heteroaryl group which may have a substituent]
   and the aforementioned fluorine-containing compound is a compound represented by the following general formula (P1):
   [Chemical Formula 3]

   R¹¹-CF₂-R¹ (P1)

   [in the formula, R¹ and R¹¹ are the same as those defined in the aforementioned general formula (S1)].
[3] The method for producing a fluorine-containing compound according to [1] above, wherein the aforementioned oxygen-containing compound is a compound represented by the following general formula (S2): 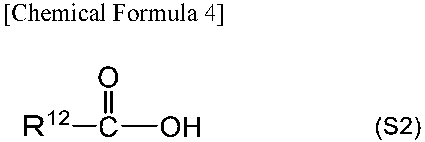
   [in the formula, R¹² is an aliphatic hydrocarbon group having 1 to 30 carbon atoms which may have a substituent, an alkoxy group which may have a substituent, an aliphatic heterocyclic group which may have a substituent, an aryl group which may have a substituent, or a heteroaryl group which may have a substituent]
   and the aforementioned fluorine-containing compound is a compound represented by the following general formula (P2): 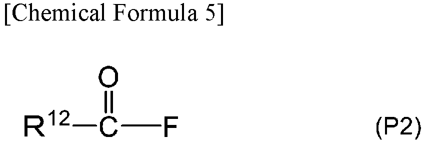
   [in the formula, R¹² is the same as defined in the aforementioned general formula (S2)].
[4] The method for producing a fluorine-containing compound according to [1] above, wherein the aforementioned oxygen-containing compound is a compound represented by the following general formula (S3):
   [Chemical Formula 6]

   R¹³-OH (S3)

   [in the formula, R¹³ is an aliphatic hydrocarbon group having 1 to 30 carbon atoms which may have a substituent]
   and the aforementioned fluorine-containing compound is a compound represented by the following general formula (P3):
      [Chemical Formula 7]

      R¹³-F (P3)
   [in the formula, R¹³ is the same as defined in the aforementioned general formula (S3)].

### Advantageous Effects of Invention

By using the method for producing a fluorine-containing compound according to the present invention, a wide variety of oxygen-containing compounds having an aldehyde group, a ketone group, a carboxy group, or a hydroxy group can be deoxofluorinated under relatively mild conditions, thereby obtaining a variety of fluorine-containing compounds.

### DESCRIPTION OF EMBODIMENTS

In the present invention and the specification of the present application, "Cₚ₁₋ₚ₂" (p1 and p2 are positive integers satisfying a relationship of p1 < p2) means a group in which the number of carbon atoms is from p1 to p2.

In the present invention and the specification of the present application, "a group which may have a substituent" includes both a group which has no substituent and a group which has a substituent.

Further, hereinafter, a "compound (n)" refers to a compound represented by a formula (n).

In the present invention and the specification of the present application, the term "C₁₋₃₀ aliphatic hydrocarbon group" includes all of a C₁₋₃₀ alkyl group which may have a substituent, a C₂₋₃₀ alkenyl group which may have a substituent, and a C₂₋₃₀ alkynyl group which may have a substituent. A "C₁₋₃₀ aliphatic hydrocarbon group" may be a linear group, a branched group, or a cyclic group.

In the present invention and the specification of the present application, a "Cₚ₁₋ₚ₂ aliphatic hydrocarbon group having a substituent" refers to a group in which one or more, preferably 1 to 3, hydrogen atoms bonded to a carbon atoms of the Cₚ₁₋ₚ₂ aliphatic hydrocarbon group have been substituted with other functional groups. When two or more substituents are present, the substituents may be the same as or different from each other.

In the present invention and the specification of the present application, a "C₁₋₃₀ alkyl group" is an alkyl group having 1 to 30 carbon atoms, and may be linear or branched, or may be a group containing a cyclic structure. Examples of the C₁₋₃₀ alkyl group include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an eicosyl group, a heneicosyl group, a docosyl group, a tricosyl group, a tetracosyl group, a pentacosyl group, a hexacosyl group, a heptacosyl group, an octacosyl group, a nonacosyl group, a triacontyl group, a cyclopropyl group, a cyclobutyl group, a cyclopentyl group, a cyclohexyl group, a cycloheptyl group, and a cyclooctyl group.

In the present invention and the specification of the present application, a "C₁₋₁₀ alkyl group" is an alkyl group having 1 to 10 carbon atoms, and may be linear or branched, or may be a group containing a cyclic structure. A "C₂₋₁₀ alkyl group" is an alkyl group having 2 to 10 carbon atoms, and may be linear or branched. Examples of the C₁₋₁₀ alkyl group include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, a heptyl group, an octyl group, a nonyl group, a decyl group, a cyclopropyl group, a cyclobutyl group, a cyclopentyl group, a cyclohexyl group, a cycloheptyl group, and a cyclooctyl group.

In the present invention and the specification of the present application, a "C₁₋₆ alkyl group" is an alkyl group having 1 to 6 carbon atoms, and may be linear or branched, or may be a group containing a cyclic structure. Examples of the C₁₋₆ alkyl group include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, a cyclopropyl group, a cyclobutyl group, a cyclopentyl group, and a cyclohexyl group.

In the present invention and the specification of the present application, a "Cₚ₁₋ₚ₂ alkyl group having a substituent" refers to a group in which one or more, preferably 1 to 3, hydrogen atoms bonded to a carbon atom of the Cₚ₁₋ₚ₂ alkyl group have been substituted with other functional groups. When two or more substituents are present, the substituents may be the same as or different from each other.

Examples of the C₂₋₃₀ alkenyl group include a group obtained by replacing at least one single bond between carbon atoms in the groups listed as the C₂₋₃₀ alkyl groups with a double bond. Specific examples of the C₂₋₃₀ alkenyl group include a vinyl group, a propenyl group, a 2-propenyl group, a butenyl group, a 1-methylpropenyl group, a 2-methylpropenyl group, a pentenyl group, a hexenyl group, a heptenyl group, an octenyl group, a nonenyl group, a decenyl group, a cyclobutenyl group, a cyclopentenyl group, and a cyclohexenyl group.

In the present invention and the specification of the present application, a "Cₚ₁₋ₚ₂ alkenyl group having a substituent" refers to a group in which one or more, preferably 1 to 3, hydrogen atoms bonded to a carbon atom of the Cₚ₁₋ₚ₂ alkenyl group have been substituted with other functional groups. When two or more substituents are present, the substituents may be the same as or different from each other.

In the present invention and the specification of the present application, a "C₁₋₃₀ alkoxy group" refers to a group in which an oxygen atom is bonded to a bonding end of an alkyl group having a linear, branched, or cyclic structure with 1 to 30 carbon atoms. Examples of an alkyl group moiety in a C₁₋₃₀ alkoxy group include the same C₁₋₃₀ alkyl groups as those described above.

In the present invention and the specification of the present application, a "C₁₋₁₀ alkoxy group" refers to a group in which an oxygen atom is bonded to a bonding end of an alkyl group having a linear, branched, or cyclic structure with 1 to 10 carbon atoms. Examples of an alkyl group moiety in a C₁₋₁₀ alkoxy group include the same C₁₋₁₀ alkyl groups as those described above.

In the present invention and the specification of the present application, a "Cₚ₁₋ₚ₂ alkoxy group having a substituent" refers to a group in which one or more, preferably 1 to 3, hydrogen atoms bonded to a carbon atom of the Cₚ₁₋ₚ₂ alkoxy group have been substituted with other functional groups. When two or more substituents are present, the substituents may be the same as or different from each other.

In the present invention and the specification of the present application, a "C₁₋₁₀ acyl group" refers to a group in which a carbonyl group is bonded to a C₁₋₉ alkyl group. An alkyl group moiety in a C₁₋₁₀ acyl group is preferably a linear or branched alkyl group having 1 to 9 carbon atoms, more preferably a linear or branched alkyl group having 1 to 6 carbon atoms, still more preferably a linear or branched alkyl group having 1 to 3 carbon atoms, and particularly preferably an acetyl group.

In the present invention and the specification of the present application, a "Cₚ₁₋ₚ₂ acyl group having a substituent" refers to a group in which one or more, preferably 1 to 3, hydrogen atoms bonded to a carbon atom of the Cₚ₁₋ₚ₂ acyl group have been substituted with other functional groups. When two or more substituents are present, the substituents may be the same as or different from each other.

In the present invention and the specification of the present application, an "aliphatic heterocyclic group" is a group containing an aliphatic heterocycle, that is, a group in which 1 to 3 carbon atoms forming a cyclic structure of an aliphatic hydrocarbon group containing the cyclic structure have been replaced with atoms other than carbon atoms. This aliphatic heterocyclic group may be a group containing a nitrogen atom (nitrogen-containing aliphatic heterocyclic group), a group containing an oxygen atom (oxygen-containing aliphatic heterocyclic group), or a group containing a sulfur atom (sulfur-containing aliphatic heterocyclic group). Further, two or more types of atoms other than carbon atoms may be constituting the aliphatic heterocyclic group. Examples of the aliphatic heterocyclic group include a pyrrolidyl group, a piperidyl group, a piperazyl group, a tetrahydrofuranyl group, a tetrahydropyranyl group, a tetrahydrothiophenyl group, a thianyl group, a morpholyl group, a thiomorpholyl group, a dioxanyl group, and a dithianyl group.

In the present invention and the specification of the present application, an "aliphatic heterocyclic group having a substituent" refers to a group in which one or more, preferably 1 to 3, hydrogen atoms bonded to a carbon atom forming the cyclic structure of the aliphatic heterocyclic group have been substituted with other functional groups. When two or more substituents are present, the substituents may be the same as or different from each other.

In the present invention and the specification of the present application, an "aryl group" refers to a cyclic group having aromaticity, and is a group in which this ring is composed only of carbon atoms.

In the present invention and the specification of the present application, a "C₆₋₁₄ aryl group" is an aromatic hydrocarbon group having 6 to 14 carbon atoms, and a C₆₋₁₂ aryl group is particularly preferred. Examples of the C₆₋₁₄ aryl group include a phenyl group, a naphthyl group, an anthryl group, and a 9-fluorenyl group, and a phenyl group is particularly preferred.

In the present invention and the specification of the present application, an "aryl group having a substituent" refers to a group in which one or more, preferably 1 to 3, hydrogen atoms bonded to a carbon atom of the aryl group have been substituted with other functional groups. When two or more substituents are present, the substituents may be the same as or different from each other. Examples of the substituent include a C₁₋₆ alkyl group, a Cₛ₋₆ alkenyl group, a C₁₋₆ alkoxy group, a C₁₋₆ alkylthio group, a halogen atom (a fluorine atom, a chlorine atom, a bromine atom, or an iodine atom), a nitro group, an aldehyde group, a C₁₋₁₀ acyl group, an amino group which may have a substituent, a C₆₋₁₄ aryl group which may have a substituent, and a C₆₋₁₄ heteroaryl group which may have a substituent. Examples of the "C₆₋₁₄ aryl group which may have a substituent" include a phenyl group, a naphthyl group, an anthryl group, a 2-methylphenyl group, a 4-methylphenyl group, a 3,5-dimethylphenyl group, a 2,6-dimethylphenyl group, a 2,4-dimethylphenyl group, a 2-trifluoromethylphenyl group, a 2-methoxyphenyl group, a 4-methoxyphenyl group, a 2,4-dimethoxyphenyl group, a 3,5-dimethoxyphenyl group, a 2-methylthiophenyl group, a 4-methylthiophenyl group, a 2,4-dimethylthiophenyl group, a 3,5-dimethylthiophenyl group, a 4-chlorophenyl group, a 4-bromophenyl group, a 4-nitrophenyl group, a 4-aminophenyl group, a 4-(methylamino)phenyl group, a 4-(dimethylamino)phenyl group, and a 4-(acetylamino)phenyl group.

In the present invention and the specification of the present application, a "heteroaryl group" refers to a cyclic group having aromaticity, and is a group in which this ring is composed of carbon atoms and atoms other than carbon atoms. The heteroaryl group may be a group containing a nitrogen atom (nitrogen-containing heteroaryl group), a group containing an oxygen atom (oxygen-containing heteroaryl group), or a group containing a sulfur atom (sulfur-containing heteroaryl group). Further, two or more types of atoms other than carbon atoms may be constituting the aromatic ring.

Examples of a C₅₋₁₄ nitrogen-containing heteroaryl group include a pyrrolyl group, an imidazolyl group, a pyrazolyl group, a pyridyl group, a pyrazinyl group, a pyrimidinyl group, a pyridazinyl group, an indolyl group, an isoindolyl group, an indolinyl group, a benzimidazolyl group, a benzotriazolyl group, a quinolyl group, an isoquinolyl group, a quinazolyl group, and a carbazolyl group. Examples of a C₅₋₁₄ oxygen-containing heteroaryl group include a furanyl group, a pyranyl group, a benzopyranyl group, and a xanthenyl group. Examples of a C₅₋₁₄ sulfur-containing heteroaryl group include a thienyl group. Examples of a C₅₋₁₄ heteroaryl group containing two or more types of heteroatoms include an oxazolyl group, an isoxazolyl group, a thiazolyl group, and an isothiazolyl group.

In the present invention and the specification of the present application, a "heteroaryl group having a substituent" refers to a group in which one or more, preferably 1 to 3, hydrogen atoms bonded to a carbon atom of the heteroaryl group have been substituted with other functional groups. When two or more substituents are present, the substituents may be the same as or different from each other. Examples of the substituent include a C₁₋₆ alkyl group, a Cₛ₋₆ alkenyl group, a C₁₋₆ alkoxy group, a C₁₋₆ alkylthio group, a C₁₋₁₀ acyl group, a halogen atom (a fluorine atom, a chlorine atom, a bromine atom, or an iodine atom), a trihalomethyl group, a nitro group, an aldehyde group, an amino group which may have a substituent, a C₆₋₁₄ aryl group which may have a substituent, and a C₆₋₁₄ heteroaryl group which may have a substituent.

In the present invention and the specification of the present application, an "aromatic group" includes both an aryl group (aromatic hydrocarbon group) which may have a substituent, and a heteroaryl group (heterocyclic group) which may have a substituent.

### <Deoxofluorinating agent>

A deoxofluorinating agent used in the present invention (hereinafter sometimes referred to as a "deoxofluorinating agent of the present invention") is a compound represented by the following general formula (E1). In this deoxofluorinating agent, an SF₅⁻ ion functions as a nucleophilic reagent for a carbon atom of a ketone group or a carbon atom bonded to a hydroxy group, thereby bonding a fluorine atom in place of an oxygen atom bonded to these carbon atoms. The deoxofluorinating agent of the present invention exhibits improved thermal stability and low pressure resistance, since this SF₅⁻ion forms a salt with a glyme-coordinated alkali metal ion. More specifically, in the deoxofluorinating agent of the present invention, SF₄ is unlikely to be eliminated even in a low pressure environment, and thermal decomposition at about 100°C is also suppressed. For this reason, in addition to allowing a deoxofluorination reaction to proceed under relatively mild conditions, structural limitations on a substrate to be deoxofluorinated are alleviated. The deoxofluorinating agent of the present invention can use a wide variety of oxygen-containing compounds having an aldehyde group, a ketone group, a carboxy group, or a hydroxy group as a substrate.
[Chemical Formula 8]

[M(G4)ₙ] [SF₅] (E1)

In the general formula (E1), M represents a cesium ion, a potassium ion, or a rubidium ion. Further, G4 represents tetraethylene glycol dimethyl ether (tetraglyme) (CAS No.: 143-24-8), and n represents an integer from 1 to 4. As the deoxofluorinating agent used in the present invention, a compound in which M is a cesium ion in the general formula (E1) ([Cs(G4)n][SF₅]) is preferred, and a compound in which M is a cesium ion and n is 2 ([Cs(G4)₂][SF₅]) is particularly preferred.

### <Method for producing fluorine-containing compound>

The method for producing a fluorine-containing compound according to the present invention is a method for producing a fluorine-containing compound by deoxofluorinating an oxygen-containing compound having an aldehyde group, a ketone group, a carboxy group, or a hydroxy group in one or more solvents selected from the group consisting of 1,3-dimethyl-3,4,5,6-tetrahydro-2(1H)-pyrimidinone (DMPU), tetraethylene glycol dimethyl ether (tetraglyme) (G4), dimethylacetamide (DMA), hexamethylphosphorous triamide (HMPA), and acetonitrile (MeCN) using the deoxofluorinating agent of the present invention. The deoxofluorinating agent of the present invention is stable at room temperature, but is easily decomposed when the temperature is raised in the solvent. In particular, the deoxofluorinating agent is decomposed even at about 60°C in tetrahydrofuran (THF) or dimethyl ether (DME), but is stable up to about 80°C in MeCN, and hardly decomposes in DMPU, G4, DMA, and HMPA at room temperature to 120°C. In the present invention, a fluorine-containing compound can be efficiently produced by using one or more solvents selected from the group consisting of DMPU, G4, DMA, HMPA, and MeCN as the solvent when carrying out a deoxofluorination reaction. The solvent used in the method for producing a fluorine-containing compound according to the present invention may be one type of solvent or a mixed solvent of two or more types of solvents.

In the method for producing a fluorine-containing compound according to the present invention, the deoxofluorination reaction can be caused to proceed by adding a compound to serve as a substrate and the deoxofluorinating agent of the present invention to one or more solvents selected from the group consisting of DMPU, G4, DMA, and HMPA, and maintaining the resulting mixture at room temperature to 120°C for a predetermined time. During the reaction, the temperature in the reaction system may be changed in a stepwise manner within a range of room temperature to 120°C. The amounts of the substrate compound and the deoxofluorinating agent of the present invention to be added to the reaction system are such that with respect to 1 mole of the substrate compound, the deoxofluorinating agent of the present invention is preferably from 0.5 to 100 molar equivalents more preferably from 0.5 to 50 molar equivalents, and still more preferably from 0.5 to 10 molar equivalents.

The substrate compound used in the method for producing a fluorine-containing compound according to the present invention is not particularly limited as long as it is an oxygen-containing compound having an aldehyde group, a ketone group, a carboxy group, or a hydroxy group. This oxygen-containing compound only needs to have at least one of an aldehyde group, a ketone group, a carboxy group, and a hydroxy group, and may have two or more of an aldehyde group, a ketone group, a carboxy group, and a hydroxy group. When a plurality of aldehyde groups, ketone groups, carboxy groups, and hydroxy groups are included, the same type of functional group may be included, or two or more types among the aldehyde groups, ketone groups, carboxy groups, and hydroxy groups may be included in combination.

The method for producing a fluorine-containing compound according to the present invention can produce, for example, a compound represented by the following general formula (P1) as a fluorine-containing compound when the oxygen-containing compound used as a substrate is a compound represented by the following general formula (S1).

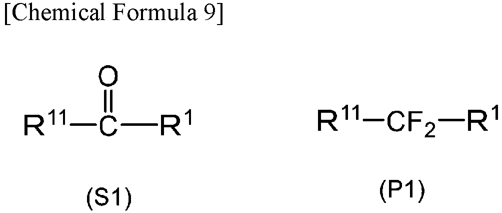

In the general formulas (S1) and (P1), R¹ is a hydrogen atom or a C₁₋₆ alkyl group. When R¹ is a C₁₋₆ alkyl group, it may be a C₁₋₆ alkyl group without a substituent, or may be a C₁₋₆ alkyl group with a substituent. This substituent is not particularly limited as long as it is a group that does not inhibit deoxofluorination by the deoxofluorinating agent of the present invention. Examples of the substituent include a C₁₋₆ alkoxy group, a C₁₋₆ alkylthio group, a halogen atom (a fluorine atom, a chlorine atom, a bromine atom, or an iodine atom), a trihalomethyl group, a nitro group, and an amino group which may have a substituent. The amino group which may have a substituent is a group in which one or two hydrogen atoms of the amino group have been substituted with a C₁₋₆ alkyl group, a C₁₋₁₀ acyl group, or the like, and specific examples thereof include a dimethylamino group, a diethylamino group, a methylamino group, an acetylamino group, and an acetylmethylamino group. As the substrate in the method for producing a fluorine-containing compound according to the present invention, in the general formula (E1), R¹ is preferably a hydrogen atom or a C₁₋₆ alkyl group having no substituent, more preferably a hydrogen atom, a methyl group, an ethyl group, a propyl group, or an isopropyl group, and still more preferably a hydrogen atom or a methyl group.

In the general formulas (S1) and (P1), R¹¹ is a C₁₋₃₀ aliphatic hydrocarbon group which may have a substituent, an alkoxy group which may have a substituent, an aliphatic heterocyclic group which may have a substituent, an aryl group which may have a substituent, or a heteroaryl group which may have a substituent.

When R¹¹ is a C₁₋₃₀ aliphatic hydrocarbon group which may have a substituent, this substituent is not particularly limited as long as it is a group that does not inhibit deoxofluorination by the deoxofluorinating agent of the present invention. Examples of the substituent include a C₁₋₆ alkoxy group, a C₁₋₆ alkylthio group, a halogen atom (a fluorine atom, a chlorine atom, a bromine atom, or an iodine atom), a trihalomethyl group, a nitro group, an amino group which may have a substituent, an aryl group which may have a substituent, and a heteroaryl group which may have a substituent. As the amino group which may have a substituent, the same groups as those exemplified above as the substituents of R¹ can be used.

When R¹¹ is a C₁₋₃₀ aliphatic hydrocarbon group which may have a substituent, this C₁₋₃₀ aliphatic hydrocarbon group which may have a substituent is preferably a C₁₋₁₀ alkyl group which may have a substituent or a C₂₋₁₀ alkenyl group which may have a substituent; more preferably a C₁₋₁₀ alkyl group which may have, as a substituent, an aryl group which may have a substituent or a heteroaryl group which may have a substituent, or a C₂₋₁₀ alkenyl group which may have, as a substituent, an aryl group which may have a substituent or a heteroaryl group which may have a substituent; and still more preferably a C₁₋₁₀ alkyl group which may have, as a substituent, a phenyl group which may have a substituent, or a C₂₋₁₀ alkenyl group which may have, as a substituent, a phenyl group which may have a substituent. Of these, a methyl group, an ethyl group, a propyl group, or a vinyl group which may have, as a substituent, a phenyl group which may have a substituent is particularly preferred. Examples of the phenyl group which may have a substituent include a phenyl group having one or more groups selected from the group consisting of a halogen atom (a fluorine atom, a chlorine atom, a bromine atom, or an iodine atom), a nitro group, an amino group, a dimethylamino group, a methylamino group, an acetylamino group, a methoxy group, a vinyl group, a propenyl group, a 1-methylvinyl group, and a hydroxy group as a substituent, and a phenyl group having no substituent.

When R¹¹ is an alkoxy group which may have a substituent, this alkoxy group is preferably a C₁₋₁₀ alkoxy group, more preferably a C₁₋₁₀ alkoxy group in which the alkyl group moiety is linear or branched, and still more preferably a C₁₋₆ alkoxy group in which the alkyl group moiety is linear.

When R¹¹ is an alkoxy group which may have a substituent, this substituent is not particularly limited as long as it is a group that does not inhibit deoxofluorination by the deoxofluorinating agent of the present invention. Examples of the substituent include a C₁₋₆ alkoxy group, a C₁₋₆ alkylthio group, a halogen atom (a fluorine atom, a chlorine atom, a bromine atom, or an iodine atom), a nitro group, an aryl group which may have a substituent, and a heteroaryl group which may have a substituent.

When R¹¹ is an alkoxy group which may have a substituent, this alkoxy group which may have a substituent is preferably a C₁₋₁₀ alkoxy group which may have a substituent, more preferably a C₁₋₁₀ alkoxy group in which the alkyl group moiety is linear or branched and which may have a substituent, and still more preferably a C₁₋₆ alkoxy group in which the alkyl group moiety is linear and which may have a substituent. Among these, a C₁₋₆ alkoxy group in which the alkyl group moiety is linear and which may have one or more substituents selected from the group consisting of a C₁₋₆ alkoxy group, a halogen atom (a fluorine atom, a chlorine atom, a bromine atom, or an iodine atom), a nitro group, and a phenyl group which may have a substituent is preferred; a C₁₋₆ alkoxy group having, as a substituent, a phenyl group which may have a substituent, a C₁₋₆ alkoxy group having, as a substituent, a phenyl group having no substituent, or a C₁₋₆ alkoxy group having no substituent is more preferred, and a C₁₋₆ alkoxy group having, as a substituent, a phenyl group having no substituent is still more preferred.

When R¹¹ is an aliphatic heterocyclic group which may have a substituent, this aliphatic heterocyclic group is preferably a nitrogen-containing aliphatic heterocyclic group, more preferably a pyrrolidyl group, a piperidyl group, a piperazyl group, or a morpholyl group, and still more preferably a pyrrolidyl group or a piperidyl group. In these nitrogen-containing aliphatic heterocyclic groups, the nitrogen atom forming the heterocycle may be protected with a protecting group. As the protecting group, those used as protecting groups for amines, such as a tert-butoxycarbonyl (Boc) group, a benzyloxycarbonyl group (Cbz group), a 9-fluorenylmethyloxycarbonyl group (Fmoc group), an allyloxycarbonyl group (Alloc group), and a 2,2,2-triethoxycarbonyl group (Troc group), can be appropriately used.

When R¹¹ is an aliphatic heterocyclic group which may have a substituent, examples of the substituent include a C₁₋₆ alkyl group, a Cₛ₋₆ alkenyl group, a C₁₋₆ alkoxy group, a C₁₋₆ alkylthio group, a halogen atom (a fluorine atom, a chlorine atom, a bromine atom, or an iodine atom), a trihalomethyl group, a nitro group, an amino group which may have a substituent, an aryl group which may have a substituent, and a heteroaryl group which may have a substituent. As the amino group which may have a substituent, the same groups as those exemplified above as the substituents of R¹ can be used.

When R¹¹ is an aliphatic heterocyclic group which may have a substituent, this aliphatic heterocyclic group which may have a substituent is preferably a nitrogen-containing aliphatic heterocyclic group which may have a substituent and in which the nitrogen atom may be protected by a protecting group; more preferably a pyrrolidyl group or piperidyl group which may have a substituent and in which the nitrogen atom may be protected by a protecting group; still more preferably a pyrrolidyl group or piperidyl group which may have a substituent and in which the nitrogen atom may be protected by a Boc group or a Cbz group; and even more preferably a pyrrolidyl group which may have a substituent and in which the nitrogen atom may be protected by a Boc group or a Cbz group.

When R¹¹ is an aryl group which may have a substituent, this aryl group is preferably a phenyl group which may have a substituent or a naphthyl group which may have a substituent, and more preferably a phenyl group which may have a substituent.

When R¹¹ is an aryl group which may have a substituent, examples of the substituent include a C₁₋₆ alkyl group, a C₂₋₆ alkenyl group, a C₁₋₆ alkoxy group, a C₂₋₆ acyl group, a halogen atom (a fluorine atom, a chlorine atom, a bromine atom, or an iodine atom), a nitro group, an amino group which may have a substituent, a hydroxy group, an aryl group, and a heteroaryl group. As the amino group which may have a substituent, the same groups as those exemplified above as the substituents of R¹ can be used.

When R¹¹ is an aryl group which may have a substituent, this aryl group which may have a substituent is preferably a phenyl group which may have a substituent, and more preferably a phenyl group which may have one or more substituents selected from the group consisting of a C₁₋₆ alkyl group, a C₂₋₆ alkenyl group, a C₁₋₆ alkoxy group, a C₂₋₆ acyl group, a halogen atom (a fluorine atom, a chlorine atom, a bromine atom, or an iodine atom), a nitro group, an amino group, a dimethylamino group, a methylamino group, an acetylamino group, a phenyl group, a halogenated phenyl group (a phenyl group substituted with a halogen atom (a fluorine atom, a chlorine atom, a bromine atom, or an iodine atom)), a pyrrolyl group, a pyrazolyl group, a pyridyl group, a pyrazinyl group, a pyrimidinyl group, an indolyl group, an isoindolyl group, a quinolyl group, an isoquinolyl group, a furanyl group, and a thienyl group.

When R¹¹ is a heteroaryl group which may have a substituent, the heteroaryl group is preferably a pyrrolyl group, a pyrazolyl group, a pyridyl group, a pyrazinyl group, a pyrimidinyl group, a pyridazinyl group, an oxazolyl group, a thiazolyl group, an imidazolyl group, an indolyl group, an isoindolyl group, a quinolyl group, an isoquinolyl group, a furanyl group, or a thienyl group, and more preferably a pyrrolyl group, a pyrazolyl group, an imidazolyl group, a pyridyl group, an indolyl group, a quinolyl group, a furanyl group, or a thienyl group.

When R¹¹ is a heteroaryl group which may have a substituent, examples of the substituent include a C₁₋₆ alkyl group, a C₂₋₆ alkenyl group, a C₁₋₆ alkoxy group, a C₂₋₆ acyl group, a halogen atom (a fluorine atom, a chlorine atom, a bromine atom, or an iodine atom), a nitro group, an amino group which may have a substituent, a hydroxy group, an aryl group, and a heteroaryl group, and among these, one or more substituents selected from the group consisting of a C₁₋₆ alkyl group, a C₂₋₆ alkenyl group, a C₁₋₆ alkoxy group, a C₂₋₆ acyl group, a halogen atom (a fluorine atom, a chlorine atom, a bromine atom, or an iodine atom), an amino group which may have a substituent, a hydroxy group, a phenyl group, and a halogenated phenyl group are preferred. As the amino group which may have a substituent, the same groups as those exemplified above as the substituents of R¹ can be used.

When R¹¹ is a heteroaryl group which may have a substituent, this heteroaryl group which may have a substituent is preferably a pyrrolyl group, a pyrazolyl group, a pyridyl group, an indolyl group, a quinolyl group, a furanyl group, or a thienyl group, and these heteroaryl groups may have a substituent. As a compound (S1), a pyrrolyl group, a pyrazolyl group, an imidazolyl group, a pyridyl group, an indolyl group, a quinolyl group, a furanyl group, or a thienyl group, which may have one or more substituents selected from the group consisting of a C₁₋₆ alkyl group, a C₂₋₆ alkenyl group, a C₁₋₆ alkoxy group, a C₂₋₆ acyl group, a halogen atom (a fluorine atom, a chlorine atom, a bromine atom, or an iodine atom), an amino group, a dimethylamino group, a methylamino group, an acetylamino group, a phenyl group, and a halogenated phenyl group, is more preferred; and a pyrrolyl group, a pyrazolyl group, an imidazolyl group, a pyridyl group, an indolyl group, a quinolyl group, a furanyl group, or a thienyl group, which may have one or more substituents selected from the group consisting of a halogen atom, a phenyl group, and a halogenated phenyl group, is still more preferred.

The method for producing a fluorine-containing compound according to the present invention can produce, for example, a compound represented by the following general formula (P2) as a fluorine-containing compound when the oxygen-containing compound used as a substrate is a compound represented by the following general formula (S2).

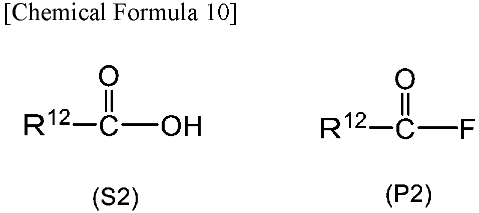

In the general formulas (S2) and (P2), R¹² is a C₁₋₃₀ aliphatic hydrocarbon group which may have a substituent, an alkoxy group which may have a substituent, an aliphatic heterocyclic group which may have a substituent, an aryl group which may have a substituent, or a heteroaryl group which may have a substituent. More specifically, as the C₁₋₃₀ aliphatic hydrocarbon group which may have a substituent, the alkoxy group which may have a substituent, the aliphatic heterocyclic group which may have a substituent, the aryl group which may have a substituent, and the heteroaryl group which may have a substituent, the same groups as those exemplified above for R¹¹ can be used, respectively, and the groups preferred above for R¹¹ are preferably used.

The method for producing a fluorine-containing compound according to the present invention can produce, for example, a compound represented by the following general formula (P3) as a fluorine-containing compound when the oxygen-containing compound used as a substrate is a compound represented by the following general formula (S3).
[Chemical Formula 11]

R¹³-OH (S3)

R¹³-F (P3)

In the general formulas (S3) and (P3), R¹³ is a C₁₋₃₀ aliphatic hydrocarbon group which may have a substituent. More specifically, as the C₁₋₃₀ aliphatic hydrocarbon group which may have a substituent, the same groups as those exemplified above for R¹¹ can be used, and the groups preferred above for R¹¹ are preferably used.

A hydroxy group in the compound (S3) may be protected by a protecting group. This protecting group can be appropriately selected and used from among known protecting groups used as protecting groups for alcohols. Specific examples of the protecting group include a trialkylsilyl group, a tetrahydropyranyl group (THP group), a methoxymethyl ether group (MOM group), a trityl group (Tr group), and a tert-butyl group. Examples of the trialkylsilyl group include a trimethylsilyl group (TMS group), a triethylsilyl group (TES group), a triisopropylsilyl group (TIPS group), and a tritert-butylsilyl group (TBS group).

### Examples

Hereinafter, the present invention will be described with reference to Examples, but the present invention is not limited to these Examples.

An NMR apparatus used in the analyses of the Examples and the like was JNM-ECS400 (400 MHz) or ECZ500R (500 MHz) manufactured by JEOL Ltd. In ¹⁹F NMR, C₆F₆ was used as a reference value of -162 PPM. The unit of yield (%) described in the Examples is mol%.

### [Production Example 1]

In the following experiments, [Cs(G4)₂][SF₅] used as a deoxofluorinating agent was synthesized in accordance with the method of Matsumoto et al. (Non Patent Document 2).

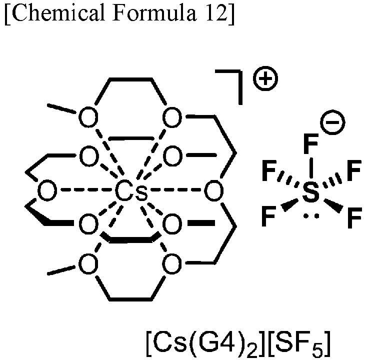

### [Example 1]

The effects of a solvent on the deoxofluorination reaction by [Cs(G4)₂][SF₅] (hereinafter sometimes referred to as "Cs(G4)₂SF₅") were examined using a silyl-protected alcohol as a substrate.

The following compound (1) was used as the silyl-protected alcohol.

G4, THF, DME, diethylene glycol dimethyl ether (CAS No.: 111-96-6) (G2), acetonitrile (MeCN), dimethylformamide (DMF), N-methylpyrrolidone (NMP), DMPU, DMA, HMPA, and dimethyl sulfoxide (DMSO) were used as solvents.

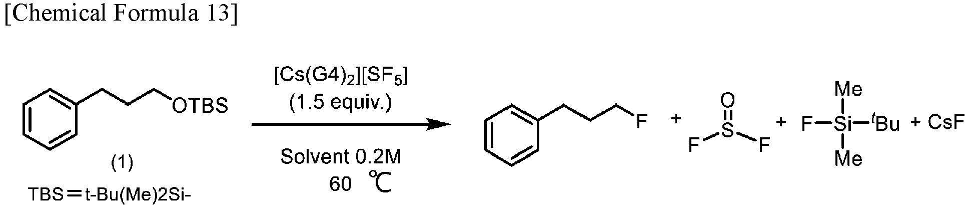

In a glove box filled with argon gas, a compound (1) (0.1 mmol) as a starting material (substrate) and 1,4-difluorobenzene (0.5 mmol, 5 µL) as an internal standard were dissolved in a solvent (0.5 mL) in a glass vial to prepare a substrate solution. In another glass vial, solid Cs(G4)₂SF₅ (0.15 mmol, 1.5 equivalents with respect to the substrate) was charged, and the above substrate solution was added using a syringe and mixed well to prepare a reaction mixture, which was a uniform solution. The obtained reaction mixture was transferred to an NMR tube containing an FEP inlet and sealed using a PTFE plug. Subsequently, the NMR tube was heated to 60°C to carry out the reaction. The progress of the reaction was monitored by ¹⁹F NMR.

**[Table 1]**

| Solvent (0.2 M) | Reaction time (hours) | Conversion (%) | NMR yield (%) |
|---|---|---|---|
| G4 | 22 | 39 | 28 |
| THF | 22 | 2 | 0 |
| DME | 22 | 4 | 0 |
| G2 | 22 | 21 | 14 |
| MeCN | 22 | 12 | 8 |
| DMF | 22 | 70 | 16 |
| NMP | 22 | 100 | 43 |
| G4 | 72 | 88 | 71 |
| NMP | 72 | 100 | 45 |
| DMA | 72 | 100 | 78 |
| DMPU | 72 | 96 | 84 |
| HMPA | 72 | 100 | 88 |
| DMSO | 72 | 100 | 30 |

Table 1 shows the reaction time for each reaction, the conversion rate (fluorination rate) (%) of the substrate compound, and the yield (%) calculated from the NMR measurement data. As shown in Table 1, in the reaction systems using DMPU, G4, DMA, and HMPA as solvents, fluorine-containing compounds were obtained with a high yield of 70% or higher after 72 hours of reaction. In addition, when MeCN was used as the solvent, although the conversion rate was low after 22 hours of reaction, the decomposition of Cs(G4)₂SF₅ did not progress and there were few by-products. In particular, DMPU was the most suitable reaction solvent for the deoxofluorination reaction using Cs(G4)₂SF₅, since few by-products were produced and the yield was very high. On the other hand, fluorine-containing compounds were not obtained in THF or DME, in which Cs(G4)₂SF₅ was easily decomposed.

### [Example 2]

Various substrate compounds were subjected to deoxofluorination reactions using Cs(G4)₂SF₅ as a deoxofluorinating agent and DMPU as a reaction solvent to synthesize fluorine-containing compounds.

In a glove box filled with argon gas, a substrate compound (0.05 mmol) and 1,4-difluorobenzene (0.5 mmol, 5 µL) as an internal standard were dissolved in DMPU (0.4 mL) in a glass vial to prepare a substrate solution. In another glass vial, solid Cs(G4)₂SF₅ (0.1 to 0.2 mmol, 2 to 4 equivalents with respect to the substrate) was charged, and the above substrate solution was added using a syringe and mixed well to prepare a reaction mixture, which was a uniform solution. The obtained reaction mixture was transferred to an NMR tube containing an FEP inlet and sealed using a PTFE plug. Subsequently, the NMR tube was heated to a predetermined temperature to carry out the reaction. The progress of the reaction was monitored by ¹⁹F NMR.

The isolation of a reaction product was performed as follows.

A starting material (0.2 mmol) was placed in a PTFE round-bottom test tube containing a stirring bar in a glove box filled with argon gas. In a glass vial, a solution obtained by dissolving Cs(G4)₂SF₅ (0.4 to 0.8 mmol, 2 to 4 equivalents with respect to the substrate) in DMPU (1.5 mL) was prepared and placed in the above round-bottom test tube. This PTFE round-bottom test tube was sealed using a screw cap to allow the reaction to proceed at a predetermined temperature for a predetermined time. Subsequently, after cooling the crude reaction solution to room temperature, water (10 mL) was added thereto, and the product was extracted using a dichloromethane solvent. The obtained organic layer was washed with water and a saturated saline solution, and dehydrated with magnesium sulfate. Furthermore, the solvent was removed by distillation to obtain a crude product. The obtained crude product was purified by silica gel column chromatography.

**[Table 2]**

| Test group | 1 | 2 | 3 | 4 |
|---|---|---|---|---|
| Substrate | 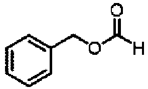 | 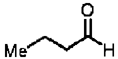 | 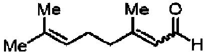 | 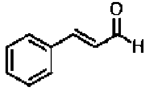 |
| Reaction product | 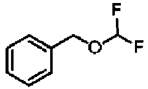 | 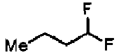 | 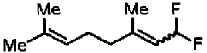 | 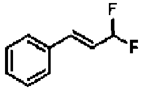 |
| Amount of Cs(G4)₂SF₅ (eq.) | 2 | 2 | 2 | 2 |
| Additive | None | None | None | None |
| Reaction conditions | 100°C, 17hours ↓ 120°C, 18hours | 60°C, 18 hours ↓ 100°C, 18 hours | 60°C, 18 hours 80°C, 72 hours | 60°C, 24 hours 80°C, 18 hours |
| NMR yield (%) | 29 | 14 | 55 | 71 |

**[Table 3]**

| Test group | 5 | 6 | 7 | 8 |
|---|---|---|---|---|
| Substrate | 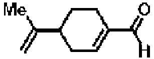 | 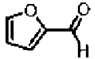 | 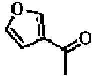 | 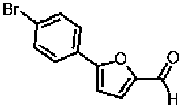 |
| Reaction product | 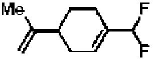 | 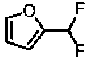 | 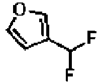 | 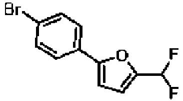 |
| Amount of Cs(G4)₂SF₅ (eq.) | 2 | 2 | 2 | 2 |
| Additive | None | None | None | None |
| Reaction conditions | 60°C, 24hours ↓ 80°C, 18hours | 60°C, 18 hours | 60°C, 18 hours ↓ 80°C, 72 hours | 60°C, 18 hours |
| NMR yield (%) | 36 | 88 | 72 | 94 |

**[Table 4]**

| Test group | 9 | 10 | 11 | 12 |
|---|---|---|---|---|
| Substrate | 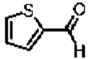 | 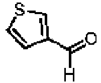 | 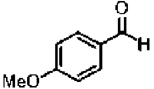 | 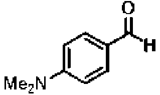 |
| Reaction product | 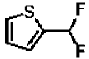 | 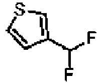 | 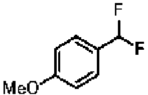 | 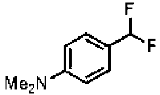 |
| Amount of Cs(G4)₂SF₅ (eq.) | 2 | 2 | 2 | 2 |
| Additive | None | None | None | None |
| Reaction conditions | 60°C, 18hours ↓ 80°C, 72hours | 60°C, 18 hours | 60°C, 18 hours | 60°C, 18 hours |
| NMR yield (%) | 86 | >95 | >95 | >95 |

**[Table 5]**

| Test group | 13 | 14 | 15 | 16 |
|---|---|---|---|---|
| Substrate | 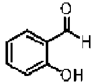 | 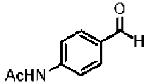 | 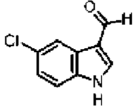 | 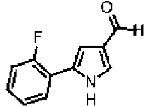 |
| Reaction product | 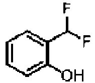 | 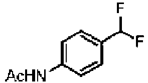 | 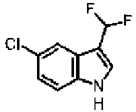 | 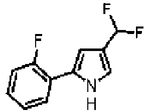 |
| Amount of Cs(G4)₂SF₅ (eq.) | 2 | 2 | 2 | 2 |
| Additive | None | None | None | None |
| Reaction conditions | 60°C, 24hours 100°C, 24hours | 60°C, 24hours 80°C, 18hours | 60°C, 18 hours ↓ 80°C, 72 hours | 60°C, 24hours ↓ 80°C, 18hours |
| NMR yield (%) | 15 | 12 | 47 | 15 |

**[Table 6]**

| Test group | 17 | 18 | 19 |
|---|---|---|---|
| Substrate | 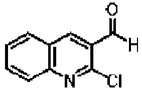 | 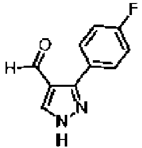 | 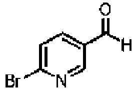 |
| Reaction product | 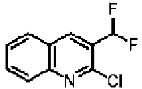 | 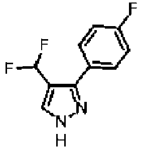 | 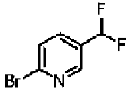 |
| Amount of Cs(G4)₂SF₅ (eq.) | 2 | 2 | 2 |
| Additive | None | None | None |
| Reaction conditions | 60°C, 24 hours ↓ 80°C, 18 hours | 60°C, 24 hours ↓ 80°C, 18 hours | 60°C, 24 hours ↓ 80°C, 18 hours |
| NMR yield (%) | 31 | 44 | 40 |

**[Table 7]**

| Test group | 20 | 21 |
|---|---|---|
| Substrate | 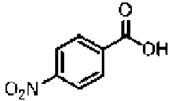 | 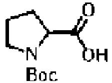 |
| Reaction product | 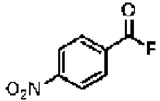 | 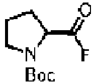 |
| Amount of Cs(G4)₂SF₅ (eq.) | 2 | 4 |
| Additive | None | None |
| Reaction conditions | r. t. 1hour | r. t. 1hour |
| NMR yield (%) | >95 | >95 |

**[Table 8]**

| Test group | 22 |
|---|---|
| Substrate | 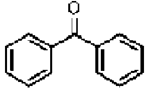 |
| Reaction product | 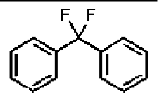 |
| Amount of Cs(G4)₂SF₅ (eq.) | 1.5 |
| Additive | None |
| Reaction conditions | 80°C, 24 hours |
| NMR yield (%) | 80 |

**[Table 9]**

| Test group | 23 | 24 |
|---|---|---|
| Substrate | 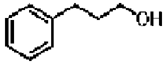 | 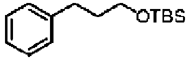 TBS= *t*-Bu(Me)₂Si- |
| Reaction product | 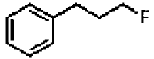 | 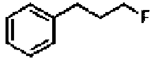 |
| Amount of Cs(G4)₂SF₅ (eq.) | 4 | 1.5 |
| Additive | 2,6-lutidine | 2,6-lutidine |
| Reaction conditions | 80°C, 48 hours | 80°C, 120 hours |
| NMR yield (%) | >95 | >95 |

### INDUSTRIAL APPLICABILITY

In the method for producing a fluorine-containing compound according to the present invention, since SF₅⁻ stabilized by forming a salt with a glyme-coordinated alkali metal ion is used as a deoxofluorinating agent, deoxofluorination can be performed under relatively mild conditions for a wide variety of oxygen-containing compounds having a ketone group, an aldehyde group, a hydroxy group, or the like. For this reason, the present invention is useful for the synthesis of a wide variety of fluorine-containing compounds.

## Claims

1. A method for producing a fluorine-containing compound,
the method comprising deoxofluorinating an oxygen-containing compound having an aldehyde group, a ketone group, a carboxy group, or a hydroxy group in one or more solvents selected from the group consisting of 1,3-dimethyl-3,4,5,6-tetrahydro-2(1H)-pyrimidinone, tetraethylene glycol dimethyl ether, dimethylacetamide, hexamethylphosphorous triamide, and acetonitrile, using a deoxofluorinating agent represented by the following general formula (E1):
[Chemical Formula 1]
[M(G4)ₙ] [SF₅] (E1)
[wherein M represents a cesium ion, a potassium ion, or a rubidium ion; G4 represents tetraethylene glycol dimethyl ether; and n represents an integer from 1 to 4] to produce a fluorine-containing compound.

2. The method for producing a fluorine-containing compound according to Claim 1, wherein said oxygen-containing compound is a compound represented by the following general formula (S1): 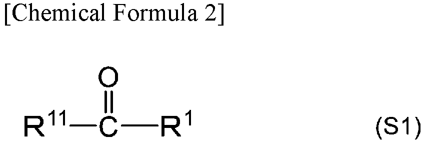
[wherein R¹ is a hydrogen atom or an alkyl group having 1 to 6 carbon atoms; and R¹¹ is an aliphatic hydrocarbon group having 1 to 30 carbon atoms which may have a substituent, an alkoxy group which may have a substituent, an aliphatic heterocyclic group which may have a substituent, an aryl group which may have a substituent, or a heteroaryl group which may have a substituent]
and said fluorine-containing compound is a compound represented by the following general formula (P1):
[Chemical Formula 3]
R¹¹-CF₂-R¹ (P1)
[wherein R¹ and R¹¹ are the same as those defined in said general formula (S1)].

3. The method for producing a fluorine-containing compound according to Claim 1, wherein said oxygen-containing compound is a compound represented by the following general formula (S2): 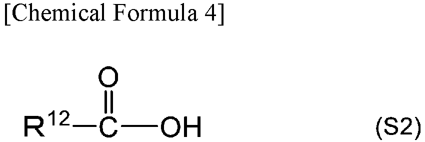
[wherein R¹² is an aliphatic hydrocarbon group having 1 to 30 carbon atoms which may have a substituent, an alkoxy group which may have a substituent, an aliphatic heterocyclic group which may have a substituent, an aryl group which may have a substituent, or a heteroaryl group which may have a substituent]
and said fluorine-containing compound is a compound represented by the following general formula (P2): 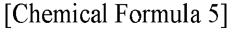 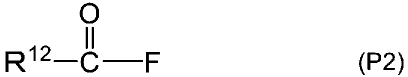
[wherein R¹² is the same as defined in said general formula (S2)].

4. The method for producing a fluorine-containing compound according to Claim 1, wherein said oxygen-containing compound is a compound represented by the following general formula (S3):
[Chemical Formula 6]
R¹³-OH (S3)
[wherein R¹³ is an aliphatic hydrocarbon group having 1 to 30 carbon atoms which may have a substituent]
and said fluorine-containing compound is a compound represented by the following general formula (P3):
[Chemical Formula 7]
R¹³-F (P3)
[wherein R¹³ is the same as defined in said general formula (S3)].
